# FASCICULE DE BREVET EUROPEEN

(11) **EP 1 032 554 B1**
(45) Date de publication et mention de la délivrance du brevet: **09.04.2003**
(21) Numéro de dépôt: 99919347.7
(22) Date de dépôt: 18.05.1999
(51) Int. Cl.: C07C 69/68, C07C 67/03, C07C 67/08, C07C 67/00, A61K 7/46

(54) **LACTATES DE CITRONELLYLE ET/OU DE DIHYDROCITRONELLYLE, LEUR PREPARATION ET LEUR UTILISATION**
CITRONNELLYL- UND/ODER DIHYDROCITRONELLYLLACTATE, IHRE HERSTELLUNG UND IHRE ANWENDUNG
CITRONELLYL AND/OR DIHYDROCITRONELLYL LACTATES, PREPARATION AND USE THEREOF

(30) Priorité: 20.05.1998 FR 9806403
(43) Date de publication de la demande: 06.09.2000
(73) Titulaire: RHODIA CHIMIE, 92408 Courbevoie Cédex (FR)
(72) Inventeur: STORET, Isabelle, F-38300 Les Eparres (FR)
(74) Mandataire: Dutruc-Rosset, Marie-Claude
(86) Numéro de dépôt international: FR9901181
(87) Numéro de publication internationale: WO99059955

(56) Documents cités:
- US-A- 3 402 721
- DATABASE CROSSFIRE [Online] Beilstein Informationssysteme GMBH,Frankfurt,DE BRN = 1712865, XP002091755 & SABATAY: BULLETIN DE LA SOCIETE CHIMIQUE DE FRANCE., vol. 47, no. 4, 1930, page 436 PARIS FR
- CHEMICAL ABSTRACTS, vol. 112, no. 11, 12 mars 1990 (1990-03-12) Columbus, Ohio, US; abstract no. 98426x, TOYOOKA KOUHEI ET AL.: "A novel and facile synthesis of 5-substituted 1,3-dioxolane-2,4-diones using trichloromethyl chloroformiate" page 742; colonne gauche; XP002091754 & TOYOOKA KOUHEI ET AL.: "A novel and facile synthesis of 5-substituted 1,3-dioxolane-2,4-diones using trichloromethyl chloroformiate" HETEROCYCLES., vol. 29, no. 5, 1989, pages 975-978, AMSTERDAM NL
- W.H.DAVIES: "Anhydrocarboxy-derivatives of Hydroxy- and Mercapto-acids" JOURNAL OF THE CHEMICAL SOCIETY.,1951, pages 1357-1359, XP002091751 LETCHWORTH GB
- G.P.BLACKBOURN ET AL.: "Studies of the Reaction of Anhydrosulphites of alpha-Hydroxycarboxylic Acids.Part 4.Anhydrosulphite Synthesis and Characterisation" JOURNAL OF THE CHEMICAL SOCIETY, SECTION C: ORGANIC CHEMISTRY.,1971, pages 257-259, XP002091752 LETCHWORTH GB
- D.J.FENN ET AL.: "Studies of the Reaction of the Anhydrosulphites of alpha-Hydroxy-carboxylic Acids. Part 4. Steric and Electronic Effects in the Reaction with Alcohols" JOURNAL OF THE CHEMICAL SOCIETY, SECTION B: PHYSICAL ORGANIC CHEMISTRY.,1970, pages 1044-1048, XP002091753 LETCHWORTH GB

## Description

La présente invention a pour objet des composés chimiques nouveaux, à savoir le lactate de citronellyle et le lactate de dihydrocitronellyle et leurs formes optiquement actives.

L'invention a trait également à leur procédé d'obtention.

La présente invention vise en particulier leur emploi dans le domaine de la parfumerie. Ledits composés possèdent des propriétés olfactives intéressantes et peuvent être employés, entre autres, pour la préparation de compositions parfumantes et de produits parfumés.

L'industrie de la parfumerie est constamment à la recherche de produits qui par l'originalité, le volume et la puissance de leur fragrance puissent conférer aux compositions dans lesquelles elles interviennent, un caractère tout à fait particulier.

On trouve dans la littérature, très peu d'informations sur l'emploi des esters lactiques en parfumerie.

Il a maintenant été trouvé que le lactate de citronellyle et le lactate de dihydrocitronellyle et leurs formes optiquement actives définies ci-après, présentaient des propriétés olfactives originales.

Il est à noter qu'il est impossible pour l'Homme du Métier de prévoir si un composé chimique donné possèdera ou non, une odeur intéréssante du point de vue olfactif et quelle sera la note.

L'invention a donc pour objet de nouveaux esters de l'acide lactique, le lactate de citronellyle et le lactate de dihydrocitronellyle répondant respectivement aux formules (I) et (I') :

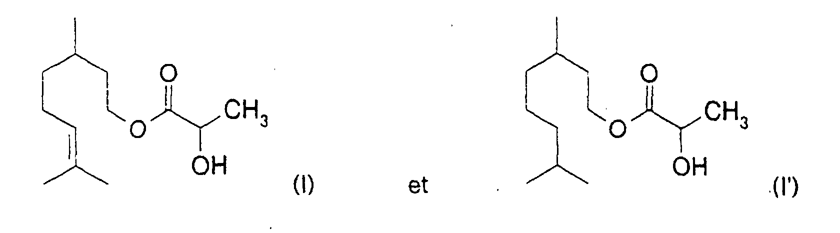

L'invention concerne aussi les formes (R) et (S) optiquement actives répondant à la formule (la) et (I'a) :

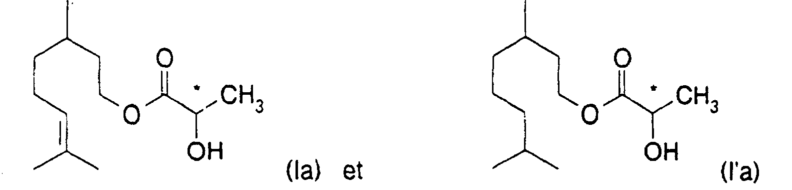

Dans les formules (la) et (l'a), l'atome de carbone optiquement actif pris en considération est l'atome de carbone en α du groupe CH₃ et de la fonction ester.

Il a été trouvé que selon la forme desdits lactates, c'est-à-dire selon qu'ils étaient sous forme racémique ou sous une forme optiquement active pure, ils présentaient une odeur différente.

Ainsi, le *(R)*-lactate de citronellyle exhale une note fleurie de type muguet, chaude et riche et plus naturelle que les alcools rosés tandis que le *(S)*-lactate de citronellyle présente une note fleurie chèvrefeuille plus proche du citronellol, ce qui lui confère un effet moins naturel que le *(R)*-lactate de citronellyle.

Quant au mélange racémique, l'odeur du lactate de citronellyle est une note fleurie de type muguet-chèvrefeuille moins puissante et moins naturelle que le *(R)*-lactate de citronellyle.

Pour ce qui est du *(R)*-lactate de dihydrocitronellyle, il présente une note citronelle, gazon, boisée alors que le *(S)*-lactate de dihydrocitronellyle une note de type rosé, boisé et citronné.

Les composés de formule (I) et (I') et leurs formes optiquement acitives (la) et (l'a) peuvent être préparés selon différents modes de préparation.

L'une des voies d'accès aux composés de formule (I) et/ou (I') consiste à faire réagir :
- l'acide lactiaue. ses sels ou esters de formule (II) : 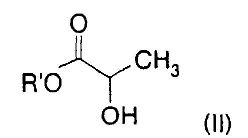 dans la formule (II), R' représente un atome d'hydrogène, un atome de métal alcalin, un radical ammonium ou un radical alkyle, linéaire ou ramifié ayant de 1 à 4 atomes de carbone,
- et le citronellol de formule (III) et/ou le dihydrocitronellol de formule (III') :

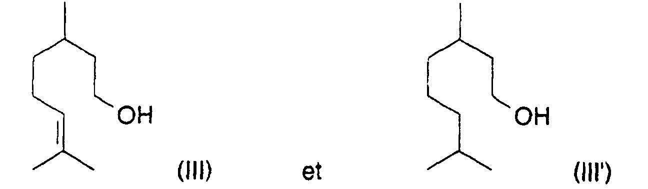

Quant à la préparation des composés optiquement actifs répondant à la formule (la) et (l'a), ils peuvent être obtenus par réaction :
- de l'acide lactique, ses sels ou esters optiquement actifs de formule (IIa) : 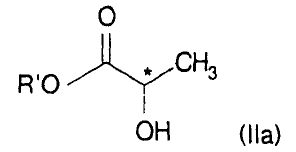 dans la formule (IIa), R' représente un atome d'hydrogène, un atome de métal alcalin, un radical ammonium ou un radical alkyle, linéaire ou ramifié ayant de 1 à 4 atomes de carbone,
- et le citronellol de formule (III) et/ou le dihydrocitronellol de formule (III').

Conformément au procédé de l'invention, on fait réagir l'acide lactique ou ses dérivés avec le citronellol et/ou le dihydrocitronellol.

Concernant les composés de formule (II) ou (IIa), on fait appel plus particulièrement à l'acide lactique, au lactate de sodium ou de potassium ou aux esters méthylique ou éthylique de l'acide lactique.

Pour préparer un ester lactique optiquement actif (la) ou (I'a), on part de l'isomère optique de formule (IIa) ayant la configuration *(R)* ou *(S)* souhaitée, sachant que la réaction permet de conserver la stéréochimie de l'isomère de départ.

On met en oeuvre préférentiellement l'acide *(R)*-lactique ou ses dérivés.

On choisit de préférence un composé de formule (IIa) ayant une bonne pureté optique, généralement moins de 10 % de l'autre énantiomère, de préférence moins de 5 %, et encore plus préférentiellement moins de 3 %, voire-même jusqu'à 0 %.

On trouve dans le commerce des composés de formule (IIa) répondant aux exigences précitées.

Ainsi, on peut faire appel à l'acide lactique du commerce qui se trouve généralement sous la forme d'une solution aqueuse à 85 %.

En ce qui concerne l'alcool, on met en oeuvre un alcool ayant une bonne pureté chimique de préférence supérieure à 85 %.

Selon la forme du composé de formule (I), (I') ou (Ia), (I'a) on peut envisager plusieurs modes de préparation.

Une première variante consiste à faire réagir l'acide lactique avec le citronellol et/ou le dihydrocitronellol. Il est également possible d'effectuer l'estérification en présence d'un solvant organique. Le solvant organique est choisi de telle sorte qu'il forme un azéotrope avec l'eau et que le point d'ébullition de son azéotrope avec l'eau soit inférieur à celui de l'alcool engagé. Comme exemples de solvants, on peut citer notamment, le toluène, le cumène ou le pseudocumène.

Une deuxième variante est de faire réagir l'acide lactique ou ses sels sous une forme activée et protégée avec le citronellol et/ou le dihydrocitronellol. Ainsi, on fait réagir d'abord l'acide lactique ou ses sels avec du phosgène, du diphosgène (trichlorométhylchloroformiate) ou du chlorure de thionyle pour obtenir respectivement le dioxolane-1,3 dione-2,4 méthyl-5 ou le propène-one-1 sulfite-1,2 sur lequel on fait réagir le citronellol et/ou le dihydrocitronellol. La quantité de phosgène, de diphosgène ou de chlorure de thionyle mise en oeuvre est généralement égale à la quantité stoechiométrique.

Une troisième variante est de conduire la réaction de l'acide lactique sous forme d'ester avec le citronellol et/ou le dihydrocitronellol, puis distillation de l'alcool correspondant libéré par transestérification.

Les différentes réactions sont conduites en présence d'un catalyseur classique de type acide. On peut citer notamment, l'acide sulfurique, l'acide p-toluène sulfonique, l'oxyde d'antimoine, les titanates d'alcoyle ou d'aryle tels que les titanates de méthyle, d'éthyle, de n-propyle, d'isopropyle. de t-butyle, de cyclohexyle, de phényle, de tolyle ; le titanate de monoéthanolamine, le titanate de diéthanolamine, le titanate de triéthanolamine.

On peut également mettre en oeuvre le méthylate de sodium ou de potassium.

La quantité des réactifs en présence est déterminée de telle sorte que le rapport molaire entre l'acide lactique et le citronellol et/ou le dihydrocitronellol varie entre 0,5 et 5, et de préférence, entre 1 et 3.

La quantité de catalyseur utilisée, exprimée par rapport au poids de l'acide lactique ou dérivé, est choisie avantageusement entre 0,1 et 5 %.

Lorsqu'il y a présence d'un solvant organique, la quantité mise en jeu peut être très variable. A titre indicatif, on peut préciser que la quantité de solvant organique peut représenter de 50 à 200 % du poids de l'acide lactique mis en jeu.

La température de la réaction est choisie de manière qu'elle soit suffisante pour permettre l'accomplissement de la réaction et la distillation de l'alcool libéré si nécessaire.

La température de la réaction est choisie de préférence entre 50 et 150°C.

La réaction est conduite avantageusement sous pression réduite avantageusement comprise entre 5 et 500 mBar.

On conduit la réaction de préférence sous atmosphère d'un gaz inerte qui peut être l'azote ou un gaz rare, de préférence l'argon.

D'un point de vue pratique, le procédé selon l'invention est simple à mettre en oeuvre.

Les différents réactifs peuvent être introduits dans n'importe quel ordre. D'une manière préférée, on choisit l'ordre des réactifs suivants : on introduit l'acide lactique ou dérivé et le citronellol et/ou le dihydrocitronellol, puis le catalyseur acide.

On porte le milieu réactionnel à la température désirée, tout en maintenant le milieu réactionnel sous agitation.

Au cours de la réaction, il y a formation d'eau ou libération de l'alcool dans le milieu réactionnel. Une variante préférée de l'invention consiste à les éliminer du milieu réactionnel, au fur et à mesure de leur formation, par tout moyen connu, notamment par distillation azéotropique.

En fin de réaction, on obtient l'ester lactique souhaité, le citronellol et/ou le dihydrocitronellol en excès et le catalyseur.

On peut récupérer l'ester lactique formé à partir du milieu réactionnel, par tout moyen approprié.

Ainsi, on peut notamment faire un lavage à l'eau suivie d'une neutralisation à l'aide d'une base.

La quantité de base, de préférence la soude est telle que le pH soit compris entre 6 et 8.

On sépare la phase organique que l'on fractionne par distillation.

On recueille le plus souvent, d'abord l'excès de citronellol et/ou le dihydrocitronellol puis l'ester lactique désiré qui peut être selon⁻ le cas, un mélange racémique de lactate de citronellyle de formule (I) et/ou de lactate de dihydrocitronellyle de formule (l') ou leurs formes optiquement actives de formule (la) ou (l'a) ou éventuellement un mélange de formes optiquement actives.

Ainsi, dans l'exposé qui suit de l'invention, le terme "ester lactique" prend en compte ses différentes variantes.

Un autre objet de la présente invention a pour objet des compositions parfumantes, produits et substances parfumantes caractérisés par le fait qu'ils comprennent à titre de principe actif ayant une influence sur l'odeur, une quantité efficace d'au moins un ester lactique de formule (I), (I'), (la) et (I'a).

Les esters de l'acide lactique et du citronellol sont particulièrement intéressants. Comme mentionné précédemment, l'ester sous forme *(R)* exhale une note fleurie de type muguet, chaude et riche tandis que la forme *(S)* présente une note fleurie chèvrefeuille.

Les différents produits de l'invention peuvent servir de base aux notes chèvrefeuille, muguet et gardénia.

Par "compositions parfumantes", on désigne des mélanges de divers ingrédients tels que solvants, supports solides ou liquides, fixateurs, composés odorants divers, etc..., dans lesquels est incorporé au moins un ester lactique de l'invention, lesquels mélanges sont utilisés pour procurer à divers types de produits finis, la fragrance recherchée.

Les bases pour parfum constituent des exemples préférés de compositions parfumantes dans lesquelles au moins un ester lactique de l'invention peut être avantageusement utilisé.

Les eaux de toilettes, les lotions après rasage, les parfums, les crèmes, les savons, les gels de bain ou de douche ou les produits déodorants ou antiperspirants, qu'ils soient sous forme de sticks ou de lotions constituent des exemples de substances ou de produits finis dans lesquels l'ester lactique de l'invention apporte sa note originale.

Ils peuvent intervenir aussi dans les shampooings et dans les produits capillaires de tout type.

Ils peuvent aussi parfumer les talcs ou poudres de toute nature.

Ils peuvent également convenir pour les désodorisants d'air ambiant ou tout produit d'entretien.

Un autre exemple de compositions dans lesquelles lesdits composés peuvent être introduits de façon avantageuse, est représenté par les compositions détergentes usuelles. Ces compositions comprennent généralement un ou plusieurs des ingrédients suivants : agents tensio-actifs anioniques, cationiques ou amphotères, agents de blanchiment, azurants optiques, charges diverses, agents anti-redéposition. La nature de ces divers composants n'est pas critique et l'ester lactique de l'invention peut être ajouté à tout type de composition détergente. Elles peuvent être introduites dans les adoucissants pour textiles, sous forme liquide ou dans les compositions déposées sur support, le plus souvent un non-tissé, destinées à être employées dans les sèche-linges.

La teneur des compositions selon l'invention en ester lactique de l'invention exprimée en pourcentage en poids dans la composition considérée dépend de la nature de ladite composition (base pour parfum ou eau de toilette par exemple) et de la puissance et de la nature de l'effet recherché au niveau produit final. Il va de soi que dans une base pour parfum la teneur en ester lactique de l'invention peut peut varier largement, par exemple supérieure à 5 % en poids et peut atteindre 90 % en poids tandis que dans un parfum, une eau de toilette ou une lotion après rasage, cette teneur pourra être très inférieure à 50 % en poids.

La teneur en ester lactique peut être dans les compositions détergentes, notamment ménagères ou dans des savons, de l'ordre de 0,01 à 2%.

Ils peuvent également intervenir dans les shampooings parfumés à raison de 0,005 à 2 % ou pour parfumer tout produit capillaire.

Ainsi la limite inférieure de la teneur en ester lactique de l'invention peut être celle qui provoque une modification perceptible à l'odorat de la fragrance ou de la note du produit fini. Dans certains cas, cette teneur minimale peut être de l'ordre de 0,001 % en poids. On peut évidemment faire appel à des teneurs non comprises dans les limites des teneurs indiquées ci-avant sans pour autant sortir du cadre de la présente invention.

Les produits de l'invention sont particulièrement avantageux en raison de leur stabilité olfactive au cours de leur application.

On donne ci-après des exemples de réalisation de l'invention.

### Exemple 1

On charge dans un ballon 156,34 g (1,5 mol) de *(R)*-lactate de méthyle, 78,62 g (0,5 mol) de citronellol et 0,6 g de titanate de butyle.

On chauffe à 90°C pendant 24 h sous courant d'azote et sous pression réduite (140 mbar), le mélange réactionnel et on soutire le méthanol libéré.

Le milieu réactionnel est lavé par 3 x 100 ml d'une solution de NaCI à 10 % en poids.

La phase organique est séchée par du sulfate de magnésium.

On distille la phase organique sous pression réduite (0,2 mbar) et on recueille entre 115°C et 118°C, 65,7 g de *(R)*-lactate de citronellyle.

Le *(R)*-lactate de citronellyle est identifié par RMN¹H et RMN¹³C qui met en évidence la présence de deux diastéréoisomères (dia A et B) à savoir *(R,R)* et *(R,S)*.
- RMN¹H = (300,13 MHz ; CDCl₃) : 0,84 (d, J = 6,5 Hz, 3H) ; 1,12 (m, 1H) ; 1,24 (m, 1H) ; 1,32 (d, J = 6,8 Hz, 3H) ; 1,42 (m, 1H + 1H) ; 1,51 (s, 3H) ; 1,59 (s, 3H) ; 1,63 (m, 1H) ; 1,90 (m, 2H) ; 2,87 (pic large, 1H) ; 4,14 (m, 1H + 2H) ; 4,99 (t, J = 7,1 Hz).
- RMN¹³C = (75,47 MHz ; CDCl₃) : 16,8, 19,3 (dia A) ; 19,4 (dia B) ; 20,3, 25,3, 25,6, 29,3 (dia A) ; 29,5 (dia B) ; 35,3 ; 36,4 (dia A) ; 36,9 (dia B) ; 64,1 ; 66,7 ; 124,4 ; 131,4 ; 175,7.

L'odeur du *(R)*-lactate de citronellyle est une note fleurie de type muguet, chaude et riche et plus naturelle que les alcools rosés.

### Exemple 2

On reproduit l'exemple 1 en utilisant le *(S)*-lactate de méthyle.

On récupère 43,77 g de *(S)*-lactate de citronellyle.

L'odeur du *(S)*-lactate de citronellyle est une note fleurie chèvrefeuille plus proche du citronellol, ce qui lui confère un effet moins naturel que le *(R)*-lactate de citronellyle.

### Exemple 3

On reproduit l'exemple 1 en utilisant du lactate de méthyle.

On récupère 50 g de lactate de citronellyle.

L'odeur du lactate de citronellyle est une note fleurie de type muguet-chèvrefeuille moins puissante et moins naturelle que le *(R)*-lactate de citronellyle.

### Exemple 4

On reproduit l'exemple 1 en mettant en oeuvre le *(R)*-lactate de méthyle et le dihydrocitronellol.

L'odeur du *(R)*-lactate de dihydrocitronellyle est une note citronelle, gazon et boisée.

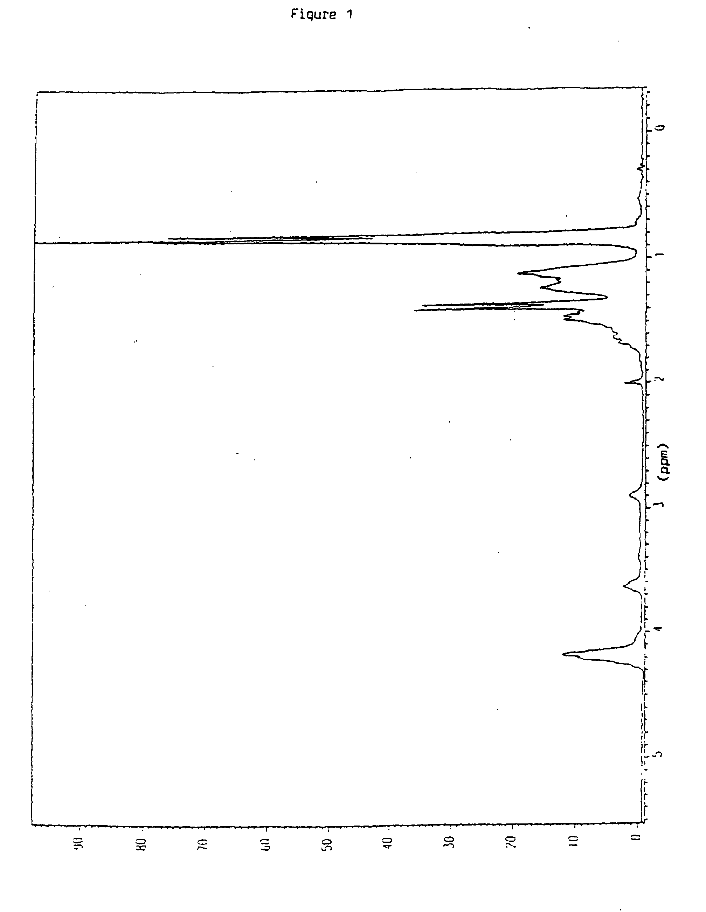

Le *(R)*-lactate de dihydrocitronellyle est identifié par RMN¹H dont le spectre constitue la figure 1.

### Exemple 5

On reproduit l'exemple 1 en mettant en oeuvre le *(S)*-lactate de méthyle et le dihydrocitronellol.

L'odeur du *(S)*-lactate de dihydrocitronellyle est une note de type rosé, boisé et citronné.

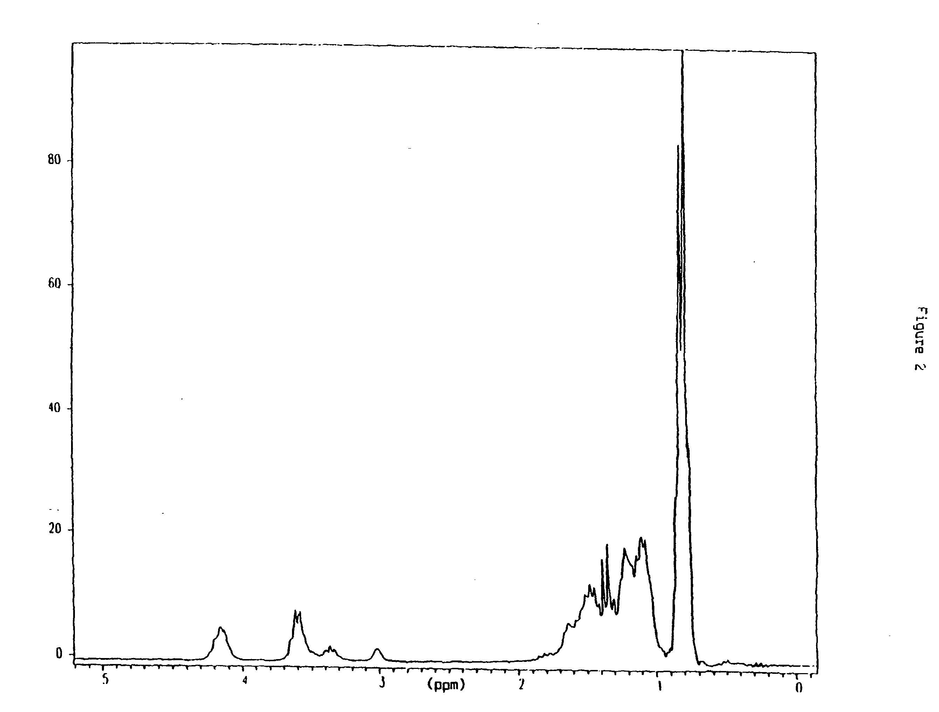

Le *(S)*-lactate de dihydrocitronellyle est identifié par RMN¹H dont le spectre constitue la figure 2.

### Exemple 6

On incorpore à 98,5 g d'une base shampooing, 1,5 g d'une solution à 33 % en poids de *(R)*-lactate de citronellyle dans du polysorbate 20 (ester de sorbitol et d'acides gras (laurique, stéarique, oléique) éthoxylé par 20 O.E.

Le shampooing a la composition pondérale suivante :
. lauryl sulfate de sodium + lauryl sulfate éthoxylé de sodium + cocoamphodiacétate de disodium + hexylène glycol (MIRACARE 2MCA S/E) 30 %
. oxyde de cocamidopropylamine et lichen extrait (ANTIPELLICULE USNATE AO) 1 %
. indian cress extrait (CAPUCINE HS) 1 %
. conservateur (GERMABEN II) 0,2 %
. acide citrique qsp pH 6,0 à 6,2
. eau déminéralisée 67,3 %
. parfum 0,5 %

Le shampoing parfumé est stable en coloration et en odeur sur une durée de 3 mois lorsqu'il est stocké à 50°C à l'abri de la lumière et lorsqu'il est stocké à 20°C à la lumière du jour.

### Exemple 7

On incorpore à 98,5 g d'une base de gel douche, 1,5 g d'une solution à 33 % en poids de *(R)*-lactate de citronellyle dans du polysorbate 20.

Le gel douche a la composition pondérale suivante :
. lauryl sulfate éthoxylé de sodium + cocoamphoacétate de sodium + cocamide MIPA (MIRACARE CS) 37,30 %
. camomille matricaire HS 2,00 %
. guar hydroxypropylé (JAGUAR C162) 0,30 %
. conservateur (GERMABEN II E) 0,20 %
. eau déminéralisée 59,7 %
. acide citrique qs pH 5,9
. parfum 0,50 %

Le gel douche parfumé est stable en coloration et en odeur sur une durée de 3 mois, lorsqu'il est stocké à 50°C à l'abri de la lumière et lorsqu'il est stocké à 20°C à la lumière du jour.

### Exemple 8

On incorpore à 99,7 g d'une base gel hydratant, 0,3 g d'une solution de *(R)*-lactate de citronellyle à 33 % en poids dans du polysorbate 20.

Le gel hydratant a la composition pondérale suivante :
. guar hydroxypropylé (JAGUAR HP 105) 0,8 %
. guar hydroxypropylé + chlorure de tri(hydroxypropyl)ammmonium (JAGUAR C162) 0,2 %
. oleth 20 ((RHODASURF ON 870) 0,05 %
. allantoine 0,2 %
. benzophénone 4 (UNIVUL MS 40) 0,5 %
. glycérol 0,25 %
. sodium PCA (NALIDONE) 2,4 %
. acide citrique 2,0 %
. hydroxyde de sodium (10%) qsp pH 8
. DMDMH hydantoin (NIPA GUARD DMDMH) 0,3 %
. parfum 0,1 %
. polysorbate 20 (TWEEN 20) 0,2 %
. eau désionisée 93 %

Le gel hydratant parfumé est stable en coloration et en odeur sur une durée de 3 mois lorsqu'il est stocké à 50°C à l'abri de la lumière et lorsqu'il est stocké à 20°C à la lumière du jour.

### Exemple 9

On incorpore 0,3 g de *(R)*-lactate de citronellyle à 99,7 g, d'une base de crème hydratante.

La base crème hydratante est préparée par mélange des deux phases suivantes de composition pondérale suivante :

### Phase A

. capryl/capric triglycéride (DERMOL M5) 4 %
. huile minérale (MARCOL 82) 2 %
. alcool stéarylique 3 %
. myristate d'isopropyle (WICKENOL 111) 2 %
. stéarate de glycéryle + polyéthylèneglycol 100 (ARLACEL 165) 6 %
. diméthicone (MIRASIL DM 300) 4 %

### Phase B

. eau désionisée 70,7 %
. glycérol 8 %
. conservateur (GERMABEN II) 0,3 %

La crème hydratante parfumée est stable en coloration et en odeur sur une durée de 3 mois lorsqu'elle est stockée à 50°C à l'abri de la lumière et lorsqu'elle est stockée à 20°C à la lumière du jour.

### Exemple 10

On incorpore 0,2 g de *(R)*-lactate de citronellyle à 99,8 g de poudre détergente.

La poudre détergente a la composition pondérale suivante :
. alkylbenzènesulfonate de sodium linéaire (LABS NANSA) 10 %
. SOAP 5 %
. alcool éthoxylé C12, 7OE (SINPERONIC A7) 2 %
. tripolyphosphate de sodium (RHODIAPHOS HPA 3,5) 25 %
. carbonate de sodium 10 %
. silicate de sodium R2 5 %
. sulfate de sodium qsp %
. carboxyméthyl cellulose sodique 1 %
. perborate de sodium 15 %
. TAED 5 %
. diéthylènetriaminepenta(méthylènephosphonique acide) (DEQUEST 2066) 1 %
. anti-mousse (RHODORSIL 20448) 1 %
. azurant optique (TINOPAL DMS) 0,2 %

La poudre détergente parfumée est stable en coloration et en odeur sur une durée de 3 mois lorsqu'elle est stockée à 50°C à l'abri de la lumière et lorsqu'elle. est stockée à 20°C à la lumière du jour.

### Exemple 11

On prépare une composition parfumante contenant :
- dihydrojasmonate de méthyle 10 g
- alcool phényléthylique 15 g
- indol en solution à 10 % dans le DEP 0,5 g de solution
- acétate de benzyle 2,5 g
- MAYOL (1-hydroxyméthyl-4-isopropylcyclohexane) 5 g
- 4-(4-hydroxy-4-méthylpentyl)-3-cyclohexène-1-carboxaldéhyde (LYRAL) 4,5 g
- p-tert-butyl--méthylhydrocinnamaldéhyde (LILIAL) 5 g
- (R)-lactate de citronellyle 7,5 g

L'utilisation de *(R)*-lactate de citronellyle permet de reconstituer une base muguet, qui procure un effet beaucoup plus naturel que la base qui n'en contient pas.

## Revendications

1. Lactates de citronellyle et/ou de dihydrocitronellyle répondant aux formules (I) et (I'):

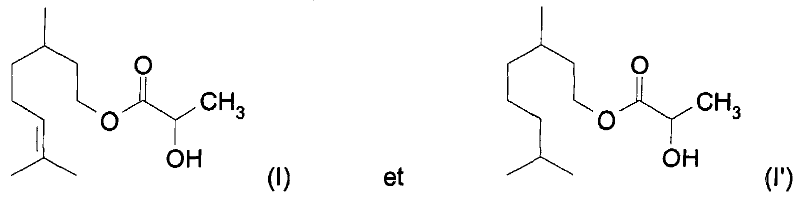

2. Lactates de citronellyle et/ou de dihydrocitronellyle, optiquement actifs sous forme *(R)* ou *(S)* répondant aux formules (la) et (I'a) :

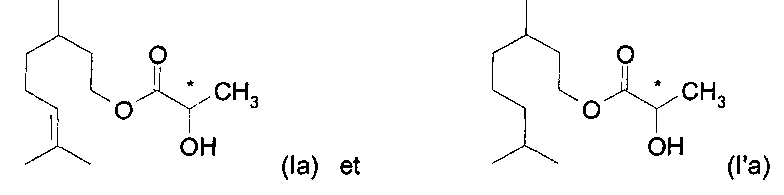

3. Procédé de préparation du lactate de citronellyle et/ou de dihydrocitronellyle répondant aux formules (I) et (I') décrit dans la revendication 1 **caractérisé par le fait qu'**il consiste à faire réagir, en présence d'un catalyseur d'estérification ou de transestérification, le citronellol et/ou le dihydrocitronellol et l'acide lactique, ses sels ou esters de formule (II) : 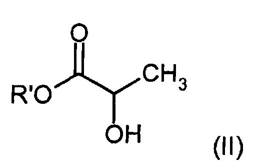 dans la formule (II), R' représente un atome d'hydrogène, un atome de métal alcalin, un radical ammonium ou un radical alkyle, linéaire ou ramifié ayant de 1 à 4 atomes de carbone.

4. Procédé de préparation du lactate de citronellyle et/ou de dihydrocitronellyle optiquement actif(s) répondant aux formules (la) et (I'a) décrits dans la revendication 2 **caractérisé par le fait qu'**il consiste à faire réagir, en présence d'un catalyseur d'estérification ou de transestérification, le citronellol et/ou le dihydrocitronellol et l'acide lactique, ses sels ou esters, optiquement actifs de formule (IIa) : 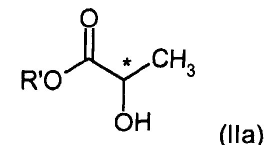 dans la formule (IIa), R' représente un atome d'hydrogène, un atome de métal alcalin, un radical ammonium ou un radical alkyle, linéaire ou ramifié ayant de 1 à 4 atomes de carbone.

5. Procédé selon l'une des revendications 3 et 4 **caractérisé par le fait que** le composé de formule (II) est l'acide lactique, le lactate de sodium ou de potassium ou les esters méthylique ou éthylique de l'acide lactique.

6. Procédé selon l'une des revendications 3 à 5 **caractérisé par le fait qu'**il consiste à faire réagir l'acide lactique ou ses dérivés avec le citronellol et/ou le dihydrocitronellol éventuellement en présence d'un solvant organique qui forme un azéotrope avec l'eau.

7. Procédé selon l'une des revendications 3 à 5 **caractérisé par le fait qu'**il consiste à faire réagir l'acide lactique ou ses sels avec du phosgène, du diphosgène (trichlorométhylchloroformiate) ou du chlorure de thionyle pour obtenir respectivement le dioxolane-1,3 dione-2,4 méthyl-5 ou le propène-one-1 sulfite-1,2 sur lequel on fait réagir le citronellol et/ou le dihydrocitronellol.

8. Procédé selon l'une des revendications 3 à 5 **caractérisé par le fait qu'**il consiste à faire réagir l'acide lactique sous forme d'ester avec le citronellol et/ou le dihydrocitronellol puis distillation de l'alcool correspondant libéré par transestérification.

9. Procédé selon l'une des revendications 4 à 8 **caractérisé par le fait que** l'on met en oeuvre l'acide *(R)*-lactique ou ses dérivés.

10. Procédé selon l'une des revendications 3 et 4 **caractérisé par le fait que** le catalyseur est choisi parmi: l'acide sulfurique, l'acide p-toluène sulfonique, l'oxyde d'antimoine, les titanates d'alcoyle ou d'aryle de préférence les titanates de méthyle, d'éthyle, de n-propyle, d'isopropyle, de t-butyle, de cyclohexyle, de phényle, de tolyle ; le titanate de monoéthanolamine, le titanate de diéthanolamine, le titanate de triéthanolamine.

11. Procédé pour l'obtention de compositions parfumantes, de substances parfumées ou de produits finis parfumés **caractérisé par le fait que** l'on ajoute aux constituants usuels de ces compositions parfumantes, de substances ou produits finis, une quantité efficace d'au moins un lactate de citronellyle ou de dihydrocitronellyle répondant à la formule (I) ou (I') décrit dans la revendication 1.

12. Procédé pour l'obtention de compositions parfumantes, de substances parfumées ou de produits finis parfumés **caractérisé par le fait que** l'on ajoute aux constituants usuels de ces compositions parfumantes, de substances ou produits finis, une quantité efficace d'au moins un lactate de citronellyle ou de dihydrocitronellyle optiquement actif répondant à la formule (la) ou (l'a) décrit dans la revendication 2.

13. Compositions parfumantes, produits et substances parfumées **caractérisés par le fait qu'**ils comprennent à titre de principe actif ayant une influence sur l'odeur une quantité efficace d'au moins un lactate de citronellyle ou de dihydrocitronellyle répondant à la formule (I) ou (I') décrit dans la revendication 1.

14. Compositions parfumantes, produits et substances parfumées **caractérisés par le fait qu'**ils comprennent à titre de principe actif ayant une influence sur l'odeur une quantité efficace d'au moins un lactate de citronellyle optiquement actif répondant à la formule (la) ou d'au moins un lactate de dihydrocitronellyle optiquement actif répondant à la formule (l'a) décrit dans la revendication 2.

15. Article parfumé selon l'une des revendications 13 et 14 sous forme de parfum, d'eau de toilette, de lotions après rasage, de parfums, de savons, de gels de bain ou de douche, de produits déodorants ou antiperspirants, de shampooings ou tout produit capillaire, de talcs ou poudres de toute nature, de désodorisants d'air ambiant, de tout produit d'entretien ou de compositions détergentes ou d'adoucissants pour textiles.

16. Utilisation du lactate de citronellyle répondant à la formule (I) décrit dans la revendication 1 comme note fleurie.

17. Utilisation du lactate de citronellyle *(R)* optiquement actif répondant à la formule (la) décrit dans la revendication 2 comme note fleurie de type muguet.

18. Utilisation du lactate de citronellyle *(S)* optiquement actif répondant à la formule (la) décrit dans la revendication 2 comme note fleurie chèvrefeuille.

19. Utilisation du lactate de dihydrocitronellyle *(R)* optiquement actif répondant à la formule (l'a) décrit dans la revendication 2 comme note citronelle, gazon, boisée.

20. Utilisation du lactate de dihydrocitronellyle *(S)* optiquement actif répondant à la formule (l'a) décrit dans la revendication 2 comme note de type rosé, boisé, citronné.

## Patentansprüche

1. Citronellyl- und/oder Dihydrocitronellyllactate der Formeln (I) und (I')

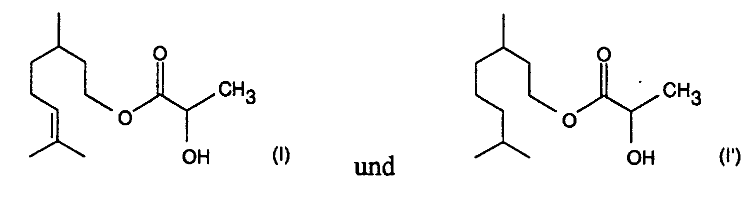

2. Optisch aktive Citronellyl- und/oder Dihydrocitronellyllactate in der (*R*)-oder (*S*)-Form der Formeln (Ia) und (I'a)

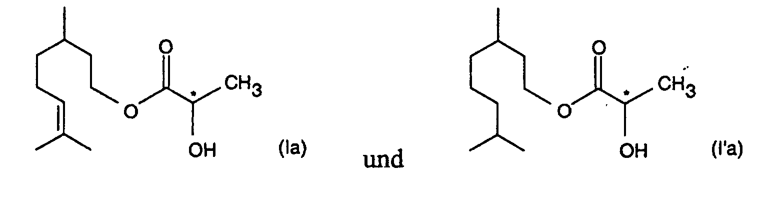

3. Verfahren zur Herstellung von Citronellyl- und/oder Dihydrocitronellyllactaten der Formeln (I) und (I'), wie in Anspruch 1 beschrieben, **dadurch gekennzeichnet, daß** man Citronellol und/oder Dihydrocitronellol mit Milchsäure, ihren Salzen oder Estern der Formel (II) 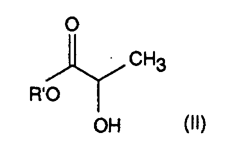 in Gegenwart eines Veresterungs- oder Umesterungskatalysators reagieren läßt, wobei in der Formel (II) R' ein Wasserstoffatom, ein Alkalimetall, einen Ammoniumrest oder einen linearen oder verzweigten Alkylrest mit 1 bis 4 Kohlenstoffatomen darstellt.

4. Verfahren zur Herstellung von optisch aktiven Citronellyl- und/oder Dihydrocitronellyllactaten der Formeln (Ia) und (I'a), wie in Anspruch 2 beschrieben, **dadurch gekennzeichnet, daß** man Citronellol und/oder Dihydrocitronellol mit optisch aktiver Milchsäure, ihren Salzen oder Estern der Formel (IIa) 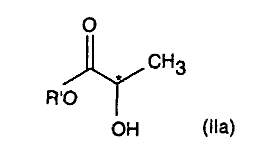 in Gegenwart eines Veresterungs- oder Umesterungskatalysators reagieren läßt, wobei in der Formel (IIa) R' ein Wasserstoffatom, ein Alkalimetallatom, einen Ammoniumrest oder einen linearen oder verzweigten Alkylrest mit 1 bis 4 Kohlenstoffatomen darstellt.

5. Verfahren nach Anspruch 3 oder 4, **dadurch gekennzeichnet, daß** die Verbindung der Formel (II) Milchsäure, Natrium- oder Kaliumlactat oder Milchsäuremethylester oder Milchsäureethylester ist.

6. Verfahren nach einem der Ansprüche 3 bis 5, **dadurch gekennzeichnet, daß** man die Milchsäure oder ihre Derivate mit dem Citronellol und/oder Dihydrocitronellol gegebenenfalls in Gegenwart eines organischen Lösemittels, welches mit Wasser ein Azeotrop bildet, reagieren läßt.

7. Verfahren nach einem der Ansprüche 3 bis 5, **dadurch gekennzeichnet, daß** man die Milchsäure oder ihre Salze mit Phosgen, Diphosgen (Trichlormethylchlorformiat) oder Thionylchlorid reagieren läßt, um 5-Methyl-1,3-dioxolan-2,4-dion oder Propen-1-on-1,2-sulfit zu erhalten, das man mit dem Citronellol und/oder Dihydrocitronellol reagieren läßt.

8. Verfahren nach einem der Ansprüche 3 bis 5, **dadurch gekennzeichnet, daß** man die Milchsäure in Form ihres Esters mit dem Citronellol und/oder Dihydrocitronellol reagieren läßt und dann den entsprechenden, durch Umesterung freigesetzten Alkohol abdestiliert.

9. Verfahren nach einem der Ansprüche 4 bis 8, **dadurch gekennzeichnet, daß** man (*R*)-Milchsäure oder ihre Derivate einsetzt.

10. Verfahren nach Anspruch 3 oder 4, **dadurch gekennzeichnet, daß** der Katalysator ausgewählt ist aus Schwefelsäure, p-Toluolsulfonsäure, Antimonoxid, Alkyl- oder Aryltitanaten, vorzugsweise Methyl-, Ethyl-, n-Propyl-, Isopropyl-, t-Butyl-, Cyclohexyl-, Phenyl-, Tolyltitanaten; Monoethanolamintitanat, Diethanolamintitanat und Triethanolamintitanat.

11. Verfahren zur Herstellung von Duftstoffzusammensetzungen, Duftstoffen oder Duftstoff-Endprodukten, **dadurch gekennzeichnet, daß** man den üblichen Bestandteilen solcher Duftstoffzusammensetzungen, Duftstoffe oder Duftstoff-Endprodukte eine wirksame Menge mindestens eines Citronellyloder Dihydrocitronellyllactats der Formel (I) oder (I'), wie in Anspruch 1 beschrieben, zusetzt.

12. Verfahren zur Herstellung von Duftstoffzusammensetzungen, Duftstoffen oder Duftstoff-Endprodukten, **dadurch gekennzeichnet, daß** man den üblichen Bestandteilen solcher Duftstoffzusammensetzungen, Duftstoffe oder Duftstoff-Endprodukte eine wirksame Menge mindestens eines optisch aktiven Citronellyl- oder Dihydrocitronellyllactats der Formel (Ia) oder (I'a), wie in Anspruch 2 beschrieben, zusetzt.

13. Duftstoffzusammensetzungen, Duftstoffprodukte und Duftstoffe, **dadurch gekennzeichnet, daß** sie als Wirkstoff mit Einfluß auf den Duft eine wirksame Menge mindestens eines Citronellyl- oder Dihydrocitronellyllactats der Formel (I) oder (I'), wie in Anspruch 1 beschrieben, enthalten.

14. Duftstoffzusammensetzungen, Duftstoffprodukte und Duftstoffe, **dadurch gekennzeichnet, daß** sie als Wirkstoff mit Einfluß auf den Duft eine wirksame Menge mindestens eines optisch aktiven Citronellyl- oder Dihydrocitronellyllactats der Formel (Ia) oder (I'a), wie in Anspruch 2 beschrieben, enthalten.

15. Duftstoffartikel nach Anspruch 13 oder 14 in Form eines Duftstoffs, eines Eau de Toilette, von After-Shave-Lotionen, Parfümen, Seifen, Bade- oder Duschgelen, desodorierenden Produkten oder Antitranspirantien, Shampoos oder beliebigen anderen Produkten für die Haarpflege, Talkum oder Pudern beliebiger Art, Desodorantien für die Raumluft und beliebigen Pflegemitteln, Waschmittelzusammensetzungen oder Weichmachern für Textilien.

16. Verwendung des Citronellyllactat der Formel (I), wie in Anspruch 1 beschrieben, als Duftnote.

17. Verwendung des optisch aktiven *(R)*- Citronellyllactats der Formel (Ia), wie in Anspruch 2 beschrieben, als Duftnote vom Typ Maiglöckchen.

18. Verwendung des optisch aktiven (*S*)- Citronellyllactats der Formel (Ia), wie in Anspruch 2 beschrieben, als Duftnote Geißblatt.

19. Verwendung des optisch aktiven *(R)*- Dihydrocitronellyllactats der Formel (I'a), wie in Anspruch 2 beschrieben, als Duftnote Zitronenkraut, Rasen oder Wald.

20. Verwendung des optisch aktiven (*S*)- Dihydrocitronellyllactats der Formel (I'a), wie in Anspruch 2 beschrieben, als Duftnote vom Typ Tau, Wald oder Zitronenduft.

## Claims

1. Citronellyl and/or dihydrocitronellyl lactates with formulae (I) and (I'):

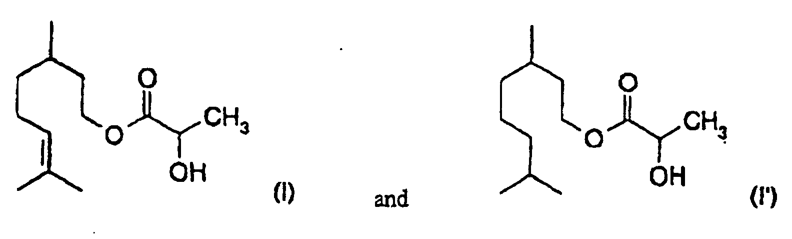

2. Optically active citronellyl and/or dihydrocitronellyl lactates in the (R) or (S) form with formulae (Ia) and Pa):

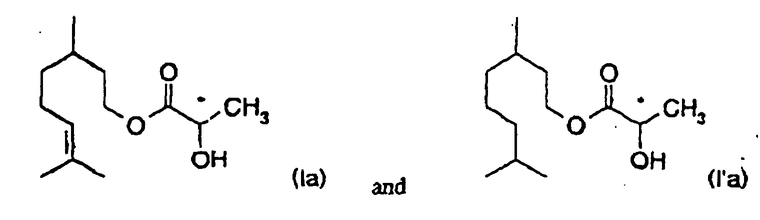

3. A process for preparing a citronellyl and/or dihydrocitronellyl lactate with formulae (I) and (I') as described in claim 1, **characterized in that** it consists of reacting, in the presence of an esterification or transesterification catalyst, citronellol and/or dihydrocitronellol and lactic acid, its salts or esters with formula (II): 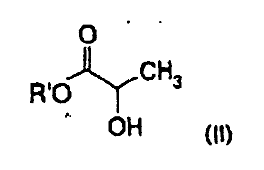 in which formula (II), R' represents a hydrogen atom, an alkali metal atom, an ammonium radical or a linear or branched alkyl radical containing 1 to 4 carbon atoms.

4. A process for preparing an optically active citronellyl and/or dihydrocitronellyl lactate with formulae (Ia) and (I'a) as described in claim 2, **characterized in that** it consists of reacting, in the presence of an esterification or transesterification catalyst, citronellol and/or dihydrocitronellol and optically active lactic acid, its salts or esters with formula (IIa): 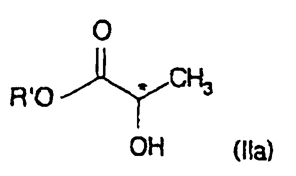 in which formula (IIa), R' represents a hydrogen atom, an alkali metal atom, an ammonium radical or a linear or branched alkyl radical containing 1 to 4 carbon atoms.

5. A process according to claim 3 or claim 4, **characterized in that** the compound with formula (II) is lactic acid, sodium or potassium lactate or methyl or ethyl esters of lactic acid.

6. A process according to one of claims 3 to 5, **characterized in that** it consists of reacting lactic acid or its derivatives with citronellol and/or dihydrocitronellol, optionally in the presence of an organic solvent that forms an azeotrope with water.

7. A process according to one of claims 3 to 5, **characterized in that** it consists of reacting lactic acid or its salts with phosgene, diphosgene (trichloromethylchloroformate) or thionyl chloride to obtain respectively 5-methyl-1,3-dioxolane-2,4-drone or propen-1-one-1,2-sulphite with which the citronellol and/or dihydrocitronellol is/are reacted.

8. A process according to one of claims 3 to 5, **characterized in that** it consists of reacting lactic acid in its ester form with citronellol and/or dihydrocitronellol then distilling the corresponding alcohol that is liberated by transesterification.

9. A process according to one of claims 4 to 8, **characterized in that** (R)-lactic aoid or a derivative thereof is used.

10. A process according to claim 3 or claim 4, **characterized in that** the catalyst is selected from: sulphuric acid, p-toluenesulphonic acid, antimony oxide, alkyl or aryl titanates, preferably methyl, ethyl, n-propyl, isopropyl, t-butyl, cyclohexyl, phenyl or tolyl titanate; monoethanolamine titanate, diethanolamine titanate and triethanolamine titanate

11. A process for producing perfuming compositions, perfumed substances or finished perfumed products, **characterized in that** an effective quantity of at least one citronellyl or dihydrocitronellyl lactate with formula (I) or (I') as defined in claim 1 is added to the usual constituents of said perfuming compositions, substances or finished products.

12. A process for producing perfuming compositions, perfumed substances or finished perfumed products, **characterized in that** an effective quantity of at least one optically active citronellyl or dihydrocitronellyl lactate with formula (Ia) or (I'a) as defined in claim 2 is added to the usual constituents of said perfuming compositions, substances or finished products.

13. Perfuming compositions, products and perfumed substances, **characterized in that** they comprise, as the active principle with an influence on the odour, an effective quantity of at least one citronellyl or dihydrocitronellyl lactate with formula (I) or (I') as defined in claim 1.

14. Perfuming compositions, products and perfumed substances, **characterized in that** they comprise, as the active principle with an influence on the odour, an effective quantity of at least one optically active citronellyl with formula (la) or at least one optically active dihydrocitronellyl lactate with formula (I'a) as defined in claim 2.

15. A perfumed article according to claim 13 or claim 14, in the form of a perfume, toilet water, after-shave lotion, perfume, soap, bath or shower gel, deodorant or antiperspirant product, shampoo or any hair care product, talc or powder of any nature, air freshener, any household product or detergent or softener for textiles.

16. Use of citronellyl lactate with formula (I) as described in claim 1, as a flowery note.

17. Use of optically active citronellyl (R) lactate with formula (Ia) as defined in claim 2, as a flowery lily-of-the-valley note.

18. Use of optically active citronellyl (S) lactate with formula (Ia) as defined in claim 2, as a flowery honeysuckle note.

19. Use of optically active (R) dihydrocitronellyl lactate with formula (I'a) as defined in claim 2, as a citrus, grassy, woody note.

20. Use of optically active (S) dihydrocitronellyl lactate with formula (l'a) as defined in claim 2, as a rosy, woody, citrus note.
